# EUROPEAN PATENT APPLICATION

(11) **EP 4 342 358 A1**
(43) Date of publication of application: **27.03.2024**
(21) Application number: 22196889.4
(22) Date of filing: 21.09.2022
(51) Int. Cl.: A61B 1/005, A61B 1/018, A61B 1/008

(54) **AN ENDOSCOPE**

(71) Applicant: Ambu A/S, 2750 Ballerup (DK)
(72) Inventor: SØRENSEN, Morten, 2750 Ballerup (DK); MATTHISON-HANSEN, Kaspar Mat, 3000 Helsingør (DK)
(74) Representative: AWA Denmark A/S

(57) **Abstract**

An endoscope where the bending section comprises a number of segments (15) interconnected by hinge members arranged in a hinge plane (B-B), said segments comprising a proximal end segment, a distal end segment and a number of intermediate segments (15). At least one of the segments (15) comprises protrusions (18) that extend inwardly from the segment wall and are adapted to radially engage and urge a working channel tube (17) into a central position around the hinge plane (B-B).

## Description

The present disclosure relates to disposable endoscopes, in particular but not exclusively to disposable EndoBronchial UltraSound endoscopes, normally abbreviated with the acronym EBUS endoscopes.

Like many other endoscopes, such EBUS endoscopes comprise a proximal handle from which an insertion cord extends. The insertion cord normally comprises a bendable main tube to the distal end of which a highly flexible bending section is connected. The bending section normally comprises a number of segments connected by hinge members so as to allow it to be articulated, that is to say bent out of a straight as-made configuration. In the straight as-made configuration the longitudinal axis of the bending section is normally aligned with the longitudinal axis of the main tube. The distal end of the bending section, in turn, is connected to a tip member with features providing desired functionalities such as illumination, vision, exit ports for tools, liquid instillation, fluid insufflation, suction etc. In EBUS endoscopes one of the features is an ultrasonic transceiver allowing to look into the tissue behind e.g. the bronchial wall. Furthermore, in EBUS endoscopes, the tip member may comprise attachment means, such as recesses, for the attachment of a balloon surrounding the ultrasonic transceiver. The balloon may be the be filled with liquid from a port in the tip part so as to provide a good match of acoustic impedance to the surrounding tissue that is to be ultrasonically probed. The proximal handle is adapted to be gripped by a hand of an operator. The handle may comprise an operating member allowing control of the bending section, so that the insertion cord may be manoeuvred during insertion into a patient e.g. into the bronchia.

In the tip member the ultrasonic transceiver emits and receives in a sideways direction i.e. at high angle transversely to the overall longitudinal direction of the endoscope, in the following understood as the centre axis of main tube when in a straight as-made configuration. That is to say, not bent by external forces. This allows the ultrasonic transceiver to look through the bronchial wall and into the tissue behind, e.g. for locating lymph nodes. The vision camera on the other hand is more forward looking, allowing the operator to manoeuvre the tip of the endoscope to a desired position in the bronchia. The working channel at the exit port in the tip is also at an angle in order to allow a tool, in particular a biopsy needle, to exit at an angle allowing penetration of the bronchial wall into lymph nodes behind the bronchial wall for sampling while at the same time being visible by the ultrasonic transceiver. The bend from the straight part of the working channel inside the main tube should have a smooth curvature into this angle of the exit port so as not to form any obstacles against advancement of the tip of the tool through the working channel and out through the exit port.

This, it will be understood, necessitates the camera and the working channel to be offset with respect to the centre axis of the main tube and the bending section. This offset must moreover be out of the plane in which the hinges are located because the articulation of the hinges must bring the ultrasonic transceiver into good engagement with the bronchial wall in order to provide a good ultrasonic image during the procedure. This, in turn, involves the drawback that there is an inherent risk of overstressing the working channel tube inside the bending section, because being out of the hinge plane the working channel tube will be compressed or stretched depending on which direction the bending section bends.

JP5361485 discloses a conventional endoscope without any ultrasonic transceiver. The exit port is aligned with the longitudinal axis of endoscope i.e. the bending section and the main tube when they are in the relaxed straight state. In the tip part the working channel is provided with an offset with respect of both of two orthogonal bending planes. Towards the bending section the offset is reduced by passing the working channel tube through a mouthpiece comprising separate annular metal insert with a number of lobes surrounding the offset passage for the insertion tube. These lobes are provided to accommodate other passing members such as leads, signal lines and heat dissipation conductors. To separate at least some of the lobes inwardly projecting protrusions are provided. The insert is provided distally of the bending section in the tip part in order to serve as fixation for the pull wires which are secured to the metal insert by soldering. This complex construction with including metal elements and soldering does not render itself for use in a disposable endoscope.

Based on this it is the object of the present disclosure to provide an endoscope having a bending section that overcomes the above drawbacks.

According to a first aspect of the present disclosure this object is achieved by an endoscope comprising a proximal handle, an insertion cord extending from said handle towards a distal end of the endoscope, where said insertion cord comprises a main tube, a tip part, and a bending section arranged between said main tube and said tip part, where the bending section comprises a number of segments interconnected by hinge members arranged in a hinge plane, said segments comprising a proximal end segment, a distal end segment and a number of intermediate segments, where the proximal end segment and at least some of said number of intermediate segments comprise pull-wire passages, where said intermediate segments each comprise a segment wall within in which the pull-wire passages are accommodated, said segment wall surrounding a central lumen so as to form generally tubular intermediate segments, wherein at least one of said segments comprises protrusions extending inwardly from said segment wall and adapted to radially engage and urge a working channel tube into a central position around the hinge plane.

Using protrusions in at least one of the intermediate segments allow the main tube to be centralized in a smooth curve utilizing parts of the bending section rather than the whole curvature having to be provided within the tip part. At the same time providing the protrusions within the bending section means that they can be easily provided as an integral part of a moulded single piece bending section directly in the moulding process for the bending section. This in turn keeps the manufacturing costs for the bending section appropriately low for a disposable endoscope.

According to a second aspect of the disclosure the object is solved by a system comprising a display device and an endoscope according to any one of the preceding claims connectable to said display device.

According to an embodiment of the first aspect of the disclosure, the protrusions comprise a number of ribs formed integrally with said segment wall. This as mentioned is advantageous as they may readily be provided in the moulding process.

According to an embodiment of the first aspect of the disclosure, the number of ribs is three. It has been found that this is ideal when having to force the working channel tube into the centralized position while at the same time still leaving room for the quite substantial cable bundles necessary for signalling to and from the multiple transducer elements forming the array of the ultrasonic transceiver in the tip of an EBUS endoscope.

According to an embodiment of the first aspect of the disclosure, the pull-wire passages are arranged in pairs in a diagonal plane perpendicular to said hinge plane. This allows efficient conversion of the pull forces on the pull-wires into torque about the hinges.

According to an embodiment of the first aspect of the disclosure, the protrusions have an angular circumferential spacing of 90°. This has been found to be sufficient to stabilize the position of the working channel tube in the central position, even in the case where there are only three protrusions as the cable bundles for the signalling may also provide support.

According to an embodiment of the first aspect of the invention, the segment is an intermediate segment, in particular but not necessarily the ultimate or the penultimate segment before the distal end segment. This has been found to provide a good curvature for the working channel tube while at the same time not overstressing the working channel tube too much during bending of the bending section.

According to an embodiment of the first aspect of the disclosure the intermediate segments comprise apertures for pull-wires, said apertures being angularly spaced from said protrusions. This helps urging the working channel tube into position around the bending plane or the bending planes if there are two orthogonal bending planes.

According to an embodiment of the first aspect of the disclosure, the tip part comprises an ultrasonic transceiver. This allows the endoscope to be provided as an EBUS endoscope, and as mentioned above allows the cable bundles necessary for signalling to and from the multiple transducer elements forming the array of the ultrasonic transceiver to be used to urge the working channel tube into the centralized position.

According to an embodiment of the second aspect of the invention the system further comprises an ultrasonic control box. This in turn allows the use of the endoscope as an EBUS endoscope. The ultrasonic control box may alternatively be denoted an ultrasound processing apparatus (UPA) operable to send electrical signals to and receive electrical signals e.g. image data from the ultrasonic transducer, and cause a display to display a live representation of the received electrical signals. The UPA may be couplable to a display and/or comprise a display. The UPA may be a portable UPA.

The display device may alternatively be denoted a video processing apparatus (VPA). The VPA may be operable to receive image data (potentially wirelessly) from one or more medical visualization devices such as from the EBUS endoscope. The VPA may be adapted to receive the image data from the one or more medical visualization devices, such as from the vision camera, and cause a display to display a live representation of the image data. The VPA may be couplable to a display and/or comprise a display. The VPA may also be portable. The UPA may be part of a VPA.

The disclosure will now be made in greater detail based on non-limiting exemplary embodiments and with reference to the schematic drawings, on which:
Fig. 1 shows a system according to the disclosure comprising an EBUS endoscope, a display device and an ultrasonic box,
Fig. 2 shows a longitudinal cross-section centrally though the distal tip part and a part of the bending section to which the tip part is attached,
Fig. 3 shows a cross-section through an intermediate section of the bending section taken along the line III-III in Fig. 2,
Fig. 4 shows an isometric view from the distal end of the bending section of Fig. 2, and
Fig. 5 shows top plan view of the bending section of Fig. 3.

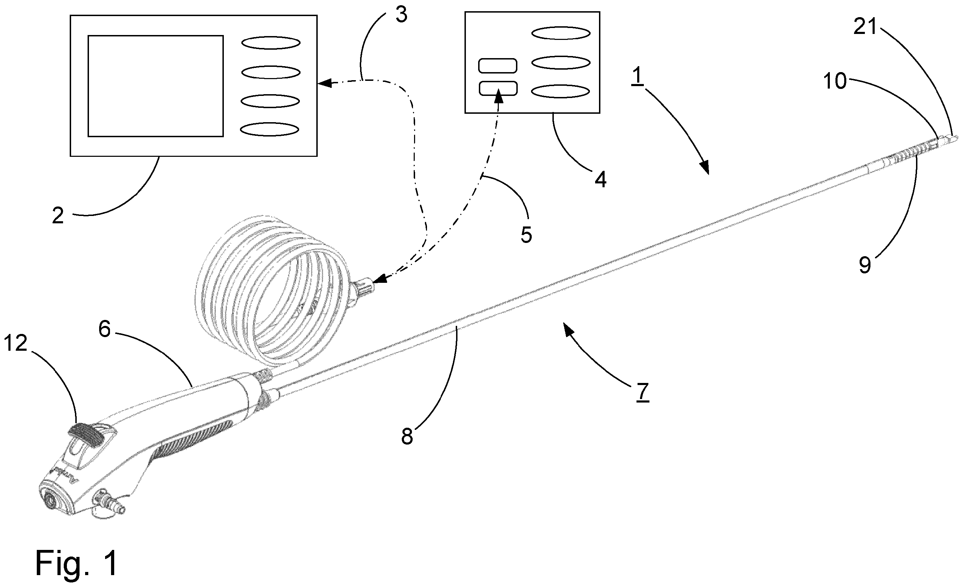

Starting with Fig. 1 an EBUS endoscope 1 according to the disclosure is shown. The endoscope 1, as shown, forms part of a system further comprising a display device 2 to which the endoscope 1 is connectable as indicated with the dashed double arrow 3. When, as envisaged, the endoscope 1 is an EBUS endoscope the system further comprises an ultrasonic box 4 to which the endoscope is also connectable, as indicated by the double arrow 5.

The endoscope 1 comprises a proximal handle 6 from which an insertion cord 7 extends towards to the distal end of the endoscope 1. The insertion cord 7 comprises several parts, such as a main tube 8, a bending section comprising a bending section body 9, and a tip part 10. Pull-wires 11 for actively bending the bending section body 9, are attached to the bending section body 9 and run from the bending section body 9 through the main tube 8 to an operating member 12 where they are also secured, thus allowing the bending section body 9 to be bent in different directions by tensioning or slacking the pull-wires 11 in a *per se* well known manner. This not only allows the distal end of the endoscope 1 to be manoeuvred to a target site but also to force the ultrasonic transceiver 21 of the tip part 10 into engagement with a tissue, such as a bronchial wall, at the target site. The bending section body 9 is an open construction and is therefore normally covered by a sheath which, however, has been entirely omitted in the figures so as not to disturb the illustration of the bending section.

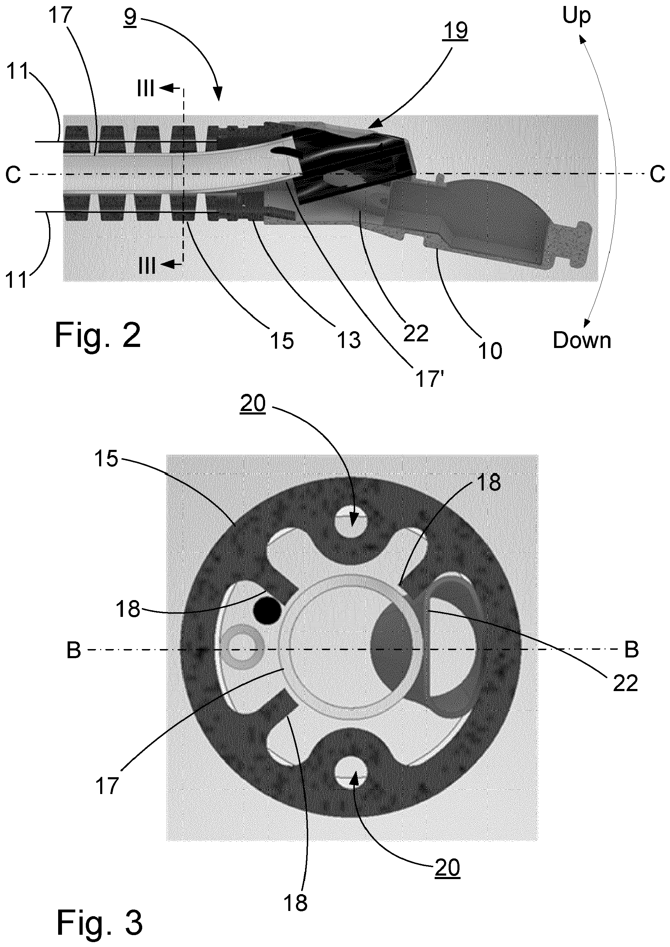

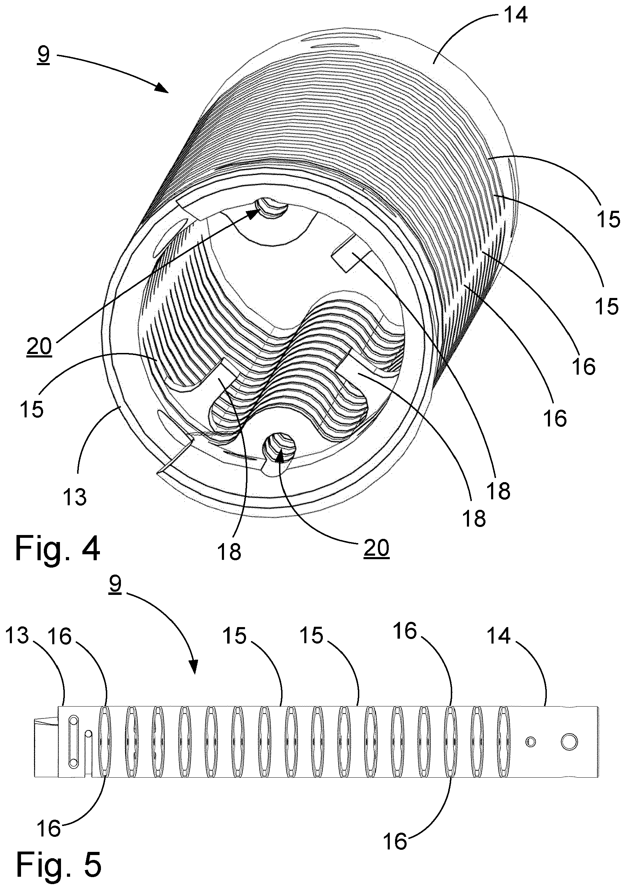

In Fig. 2 an enlarged view of a cross-section of the distal end of the endoscope 1 is shown. Some parts such as the ultrasonic transceiver 21 and an illumination and image receiver module have been omitted. The distal end comprises a tip part 10 formed by a housing adapted to accommodate *inter alia* the ultrasonic transceiver 21 and the illumination and image receiver module. The tip part 10 is secured to an articulated bending section body 9, which is preferably integrally moulded as a number of segments 13, 14, 15 interconnected by integrally formed hinge members 16 preferably comprising pairs of hinges as can be seen in Figs. 4 and 5. The hinge members 16 lie in a common plane B-B coincident with the central axis C-C of the endoscope, that is to say the centre axis of the bending section body 9 and the main tube 8 when they are in their relaxed as-made state without influence from external forces. The segments 13, 14, 15 comprise a proximal end segment 14, a distal end segment 13 and a number of intermediate segments 15. The segments 13, 14, 15 preferably all comprise passages 20 for pull-wires 11 although it may be envisaged that pull-wires 11 are terminated at an intermediate segment 15 rather than at the distal end segment 13.

Pulling the one of the pull-wires 11 will make the hinges members 16 bend and curve the bending section body 9 out of the plane B-B as indicated with the double arrow Up-Down in Fig. 2. This in turn would mean that the working channel tube 17, were it to be offset, i.e. above or below the hinge plane in Fig. 2 would be undesirably stressed by either stretching or compression during bending of the bending section body 9. This can be mitigated by centring the working channel tube so that it lies symmetrically on either side of the hinge plane, ideally concentric with the centre axis C-C as shown. However, as explained this goes against the need to offset and curve the distal end 17' of the working channel tube 17 in the tip part 10 in order to align it with an exit port 19, e.g. formed integrally with the tip part 10, as shown in Fig. 2, or by the working channel tube 17 itself. According to the disclosure the bending section body 9 has been provided with means for urging the working channel tube 17 into the desired central location. Providing such means in the bending section rather than e.g. in the tip part itself has been found to suffice. This may shorten the length of the rigid inflexible tip part 17 and facilitate manoeuvring when inserting the insertion cord 7 in body cavities such as bronchia. Furthermore, it may reduce complexity of the tip part 10 and make the tip part 10 less costly to manufacture.

As can be seen from Figs. 3 and 4 these means for urging the working channel tube 17 into the central location may comprise radially inward protrusions 18, such as ribs extending longitudinally along the inner circumferential wall of a segment 15 of the bending section body. Preferably, the segment comprising these protrusions is the ultimate or penultimate intermediate segment 15 before the distal end segment 16, but in principle they could also be formed in the distal end segment. They could evidently also be provided in a more proximal intermediate segment 15 in order to obtain a larger radius of curvature, but this would then involve a longer uncentered length of working channel tube 17 prone to stress during bending of the bending section body 9.

These protrusions 18 may be provided directly as an integral part of the bending section body 9 during injection moulding thereof without adding much complexity to the moulding process and moulding tools.

As can be seen there are three protrusions arranged with an angular spacing of 90° and with an angular offset of 45° with respect to the pull wire passages 20. Depending on the actual use for which the endoscope may be designed and dedicated there could be a different number of protrusions, in particular four with the same 90° mutual spacing and angular offset of 45° to the pull wire passages 20. In the illustrated embodiment, however, there are only three in order to make room for the cable bundle for the signals to and from the array in ultrasonic transceiver in the tip part 10. The cable bundle is illustrated only by an external surrounding sheath 22, but will comprise a substantial number wires within the sheath 22. The number will depend on the number of transducer elements, such as piezo electric elements, in the array in the ultrasonic transceiver, but figures such as 64 or 128 are not unrealistic. The cable bundle in the sheath 22 may, as will be understood from Fig. 3, at least to some extent substitute a fourth rib in urging the working channel tube into the central position.

It will be clear that providing protrusions 18 in the form of ribs as shown is not the only solution. It can be envisaged that the protrusions 18 may be wider in the circumferential direction, that they may be more trapezoid in cross-section than the almost rectangular cross-section shown, that they may be asymmetrical in cross-section, and that they may have flat, convex or concave surfaces to abut against the working channel tube 17. In particular the protrusions 18, even if referred to as ribs may have the same or shorter extension in the longitudinal direction than in the circumferential direction.

## Claims

1. An endoscope comprising a proximal handle, an insertion cord extending from said handle towards a distal end of the endoscope, where said insertion cord comprises a main tube, a tip part, and a bending section arranged between said main tube and said tip part, where
the bending section comprises a number of segments interconnected by hinge members arranged in a hinge plane, said segments comprising a proximal end segment, a distal end segment and a number of intermediate segments, where
the proximal end segment and at least some of said number of intermediate segments comprise pull-wire passages, where
said intermediate segments each comprise a segment wall within in which the pull-wire passages are accommodated, said segment wall surrounding a central lumen so as to form generally tubular intermediate segments,
wherein at least one of said segments comprises protrusions extending inwardly from said segment wall and adapted to radially engage and urge a working channel tube into a central position around the hinge plane.

2. An endoscope according to claim 1, wherein said protrusions comprise a number of ribs formed integrally with said segment wall.

3. An endoscope according to claim 2, wherein said number of ribs is three.

4. An endoscope according to any one of the preceding claims, wherein the pull-wire passages arranged in pairs in a diagonal plane perpendicular to said hinge plane.

5. An endoscope according to any one of the preceding claims wherein the protrusions have an angular circumferential spacing of 90°.

6. An endoscope according to claim 5, wherein said at least one segment is an intermediate segment.

7. An endoscope according to claim 6, wherein said at least one segment is the penultimate intermediate segment before the distal end segment.

8. An endoscope according to any one of the preceding claims wherein the intermediate segments comprise apertures for pull-wires, said apertures being angularly spaced from said protrusions.

9. An endoscope according to any one of the preceding claims wherein the tip part comprises an ultrasonic transceiver.

10. A system comprising a display device and an endoscope according to any one of the preceding claims connectable to said display device.

11. A system according to claim 10, further comprising an ultrasonic control box.
